# FASCICULE DE BREVET EUROPEEN

(11) **EP 2 989 079 B1**
(45) Date de publication et mention de la délivrance du brevet: **15.08.2018**
(21) Numéro de dépôt: 14718430.3
(22) Date de dépôt: 18.04.2014
(51) Int. Cl.: C07C 323/12, C08G 18/38

(54) **COMPOSÉ POLYOL SOUFRÉ UTILISABLE COMME MONOMÈRE POUR LA SYNTHÈSE DE POLYURÉTHANE**
SCHWEFELHALTIGE POLYOLVERBINDUNG ALS MONOMER ZUR POLYURETHANSYNTHESE
SULPHUR-COMPRISING POLYOL COMPOUND USABLE AS MONOMER FOR POLYURETHANE SYNTHESIS

(30) Priorité: 26.04.2013 FR 1353814
(43) Date de publication de la demande: 02.03.2016
(73) Titulaire: Compagnie Générale des Etablissements Michelin, 63000 Clermont-Ferrand (FR)
(72) Inventeur: FEDURCO, Milan, F-63040 Clermont-Ferrand Cedex 9 (FR); RIBEZZO, Marco, F-63040 Clermont-Ferrand Cedex 9 (FR)
(74) Mandataire: Desbordes, Guillaume
(86) Numéro de dépôt international: PCT/EP2014/058001
(87) Numéro de publication internationale: WO 2014/173839

(56) Documents cités:
- EP-A2- 0 193 303
- US-A1- 2010 021 676
- ARNOLDI A ET AL: "ISOVANILLYL SWEETENERS. SYNTHESIS AND SWEET TASTE OF SULFUR HETEROCYTES", JOURNAL OF THE CHEMICAL SOCIETY, PERKIN TRANSACTIONS 1, ROYAL SOCIETY OF CHEMISTRY, GB, no. 12, 1 janvier 1993 (1993-01-01), pages 1359-1366, XP001012752, ISSN: 0300-922X, DOI: 10.1039/P19930001359
- ARNOLD T PETERS ET AL: "1,4-Dihydroxyanthraquinone-2-thioethers. Dyes for Synthetic-polymer Fibres", JOURNAL OF THE SOCIETY OF DYERS AND COLOURISTS, vol. 93, 1 janvier 1977 (1977-01-01), pages 373-378, XP055123590,
- NEGISHI O ET AL: "Inhibition of enzymatic browning and protection of sulfhydryl enzymes by thiol compounds", PHYTOCHEMISTRY, PERGAMON PRESS, GB, vol. 54, no. 5, 1 juin 2000 (2000-06-01), pages 481-487, XP027253673, ISSN: 0031-9422 [extrait le 2000-06-01]
- FLOYD M B ET AL: "PROSTAGLANDINS AND CONGENERS. 22.1 SYNTHESIS OF 11-SUBSTITUTED DERIVATIVES OF 11-DEOXYPROSTAGLANDINS E1 AND E2. POTENTIAL BRONCHODILATORS", JOURNAL OF MEDICINAL CHEMISTRY, AMERICAN CHEMICAL SOCIETY, US, vol. 23, 1 janvier 1980 (1980-01-01), pages 903-913, XP000955991, ISSN: 0022-2623, DOI: 10.1021/JM00182A018

## Description

### 1. DOMAINE DE L'INVENTION

La présente invention est relative aux monomères susceptibles d'être utilisés pour la synthèse de polymères à unités uréthane (ou polyuréthanes) notamment destinés à des systèmes adhésifs pour collage de verre ou de métal à du caoutchouc.

Elle est plus particulièrement relative aux monomères ci-dessus du type polyols soufrés, notamment destinés à la synthèse de polyuréthanes utilisés dans les composites du type métal/ caoutchouc pour articles en caoutchouc tels que des pneumatiques.

### 2. ETAT DE LA TECHNIQUE

Les composites du type métal / caoutchouc, en particulier pour pneumatiques, sont bien connus. Ils sont généralement constitués d'une matrice en caoutchouc insaturé généralement diénique, réticulable au soufre, comportant des éléments de renforcement (ou « renforts ») métalliques tels que des fils, films ou câbles en acier au carbone.

Soumis à des contraintes très importantes lors du roulage des pneumatiques, notamment à des compressions, flexions ou variations de courbure répétées, ces composites doivent de manière connue satisfaire à un grand nombre de critères techniques, parfois contradictoires, tels qu'uniformité, flexibilité, endurance en flexion et en compression, résistance à la traction, à l'usure et à la corrosion, et maintenir ces performances à un niveau très élevé aussi longtemps que possible.

On comprend aisément que l'interphase adhésive entre caoutchouc et renforts joue un rôle prépondérant dans la pérennité de ces performances. Le procédé traditionnel pour relier les compositions de caoutchouc à de l'acier au carbone consiste à revêtir la surface de l'acier avec du laiton (alliage cuivre-zinc), la liaison entre l'acier et la matrice de caoutchouc étant assurée par sulfuration du laiton lors de la vulcanisation ou cuisson du caoutchouc. Pour améliorer l'adhésion, on utilise en outre généralement, dans ces compositions de caoutchouc, des sels organiques ou des complexes de métal tels que des sels de cobalt, en tant qu'additifs promoteurs d'adhésion.

Or, on sait que l'adhésion entre l'acier au carbone et la matrice de caoutchouc est susceptible de s'affaiblir au cours du temps, du fait de l'évolution progressive des sulfures formés sous l'effet des différentes sollicitations rencontrées, notamment mécaniques et/ou thermiques, le processus de dégradation ci-dessus pouvant être accéléré en présence d'humidité.

D'autre part, l'utilisation de sels de cobalt rend les compositions de caoutchouc plus sensibles à l'oxydation et au vieillissement, et en augmente significativement le coût, sans compter qu'il est souhaitable de supprimer à terme l'emploi de tels sels de cobalt dans les compositions de caoutchouc, en raison de l'évolution récente de la réglementation européenne sur ce type de sels métalliques.

Pour toutes les raisons exposées ci-dessus, les fabricants de composites métal / caoutchouc, en particulier les manufacturiers de pneumatiques, sont à la recherche de solutions adhésives nouvelles pour faire coller les renforts métalliques aux compositions de caoutchouc, tout en palliant, au moins en partie, les inconvénients précités.

US 2010/021676 A1 divulgue une colle à base de polyurée pour faire adhérer deux parties l'une à l'autre, comme des compositions de caoutchouc ou du métal et du caoutchouc.

### 3. BREVE DESCRIPTION DE L'INVENTION

Or, au cours de leurs recherches, les Demanderesses ont trouvé un polymère à unités uréthane nouveau issu d'au moins un composé polyol, de type soufré, répondant à un tel objectif.

Selon l'invention, ledit composé polyol soufré de départ répond à la formule (I) tel qu'énoncé dans la revendication 1.

Grâce à ce composé polyol soufré de formule (I) comportant en particulier, outre ses deux fonctions alcool primaire (diol primaire), d'une part au moins une fonction alcool secondaire et d'autre part une fonction thioéther en position alpha de cette fonction alcool, il s'est avéré possible de préparer un polyuréthane selon l'invention qui, utilisé comme primaire d'adhésion sur des renforts métalliques, donne à ces renforts l'avantage majeur et inattendu de pouvoir adhérer à des matrices de caoutchouc insaturé en utilisant de simples colles textiles telles que des colles « RFL » (résorcinol-formaldéhyde-latex) ou autres compositions adhésives équivalentes, ou encore directement (c'est-à-dire sans emploi de telles colles) à ces matrices de caoutchouc insaturé lorsque ces dernières contiennent des élastomères insaturés fonctionnalisés appropriés tels que par exemple des élastomères époxydés.

L'invention concerne également l'utilisation d'au moins un tel composé polyol de formule (I) pour la fabrication d'un polymère à unités uréthane

L'invention concerne également tout procédé de synthèse d'un polymère à unités uréthane par polycondensation d'au moins un tel composé polyol de formule (I) avec un composé polyisocyanate.

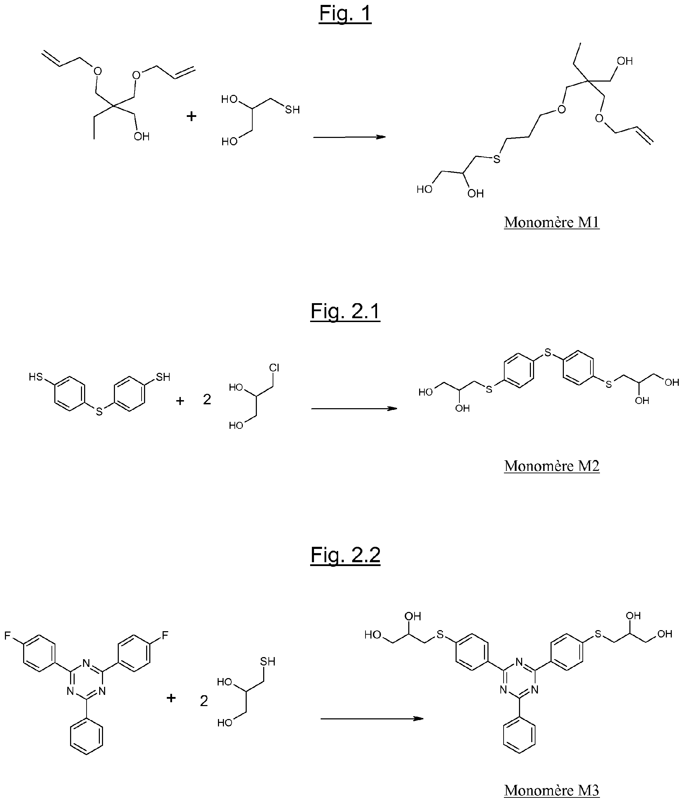

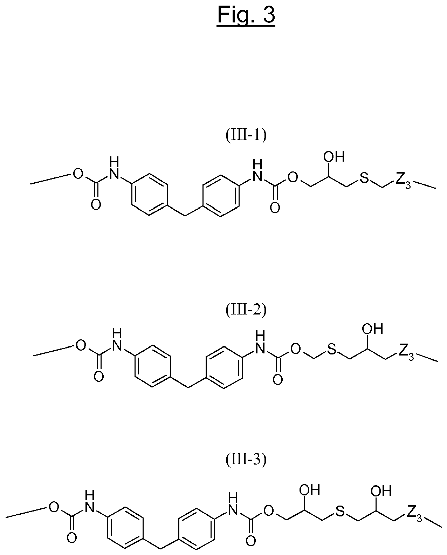

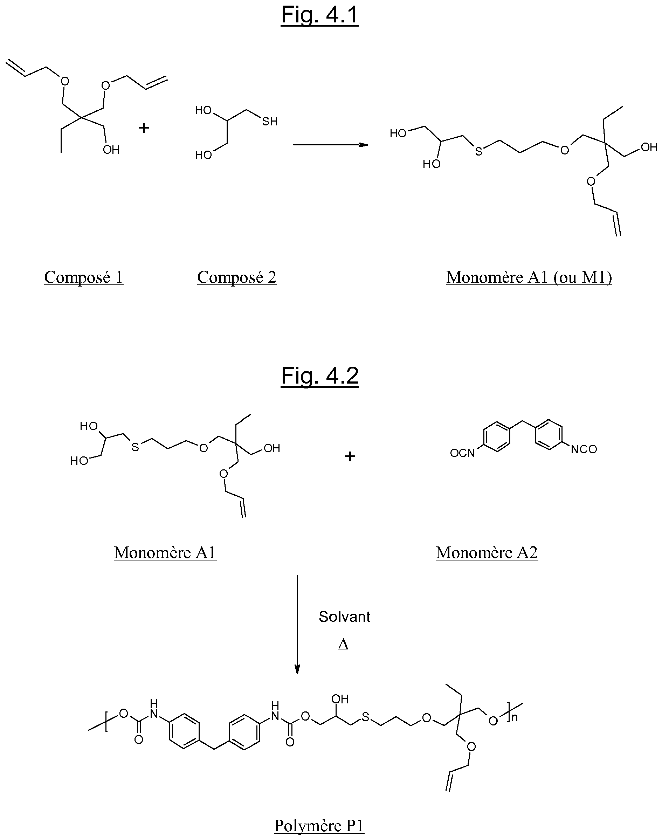

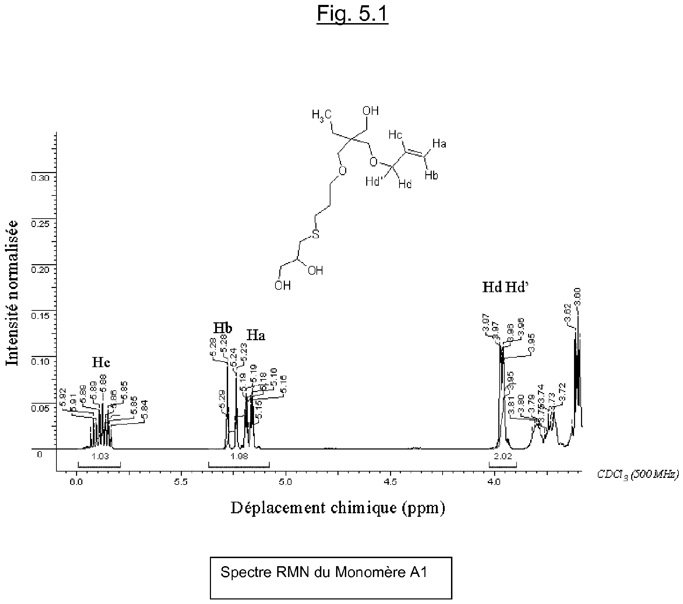

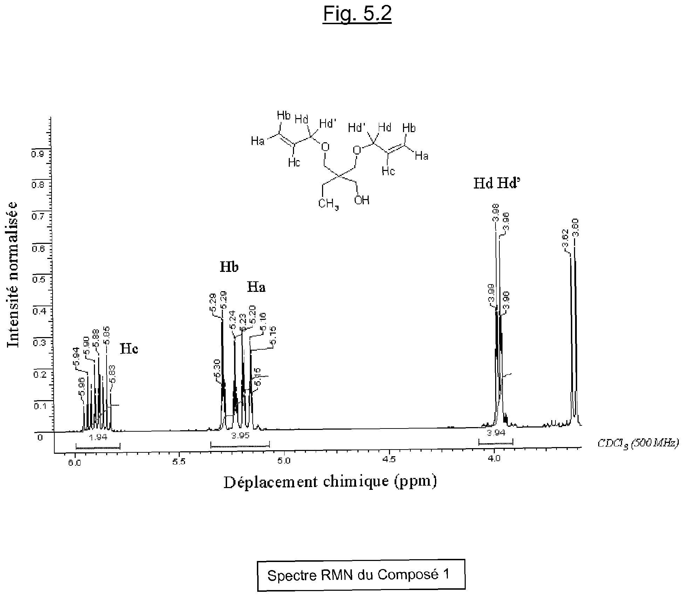

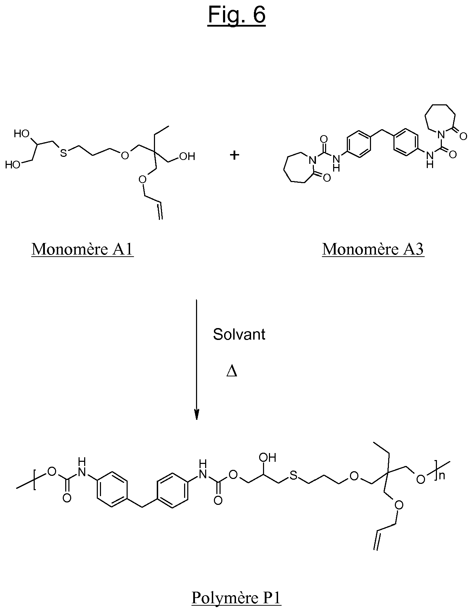

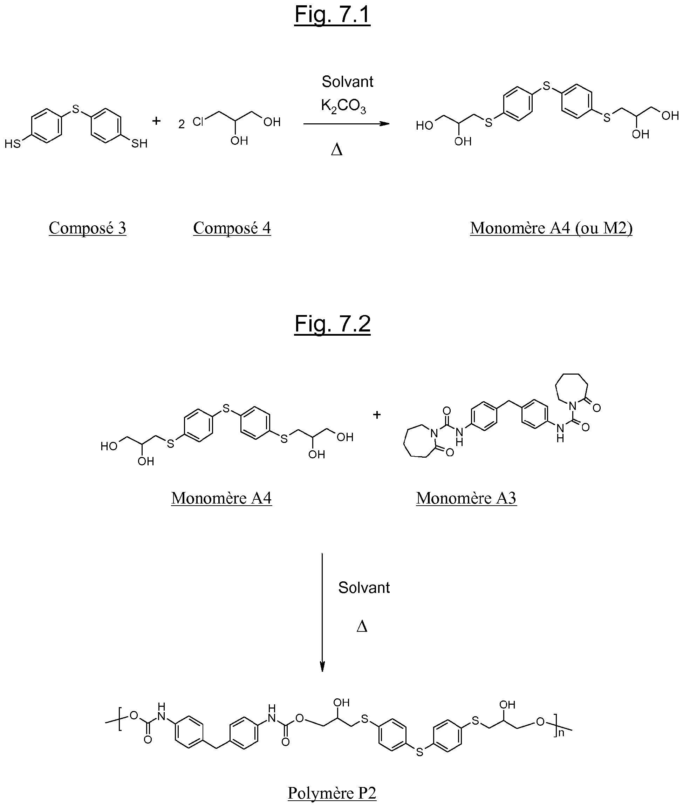

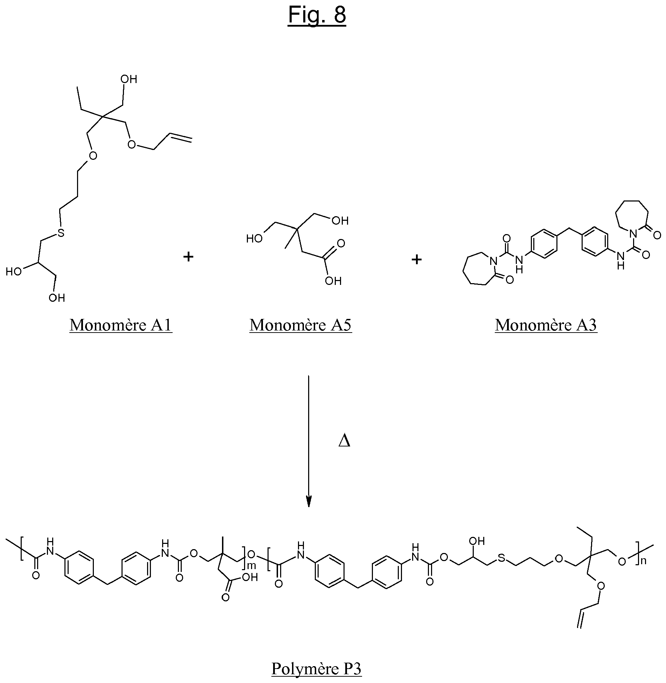

L'invention ainsi que ses avantages seront aisément compris à la lumière de la description détaillée et des exemples de réalisation qui suivent, ainsi que des figures 1 à 8 relatives à ces exemples qui représentent ou schématisent :
- des exemples de composés polyols soufrés de formule (I) (notés Monomères M1 à M3), ainsi qu'un schéma de synthèse utilisable pour l'obtention de chacun de ces composés (Fig. 1, Fig. 2.1 et 2.2) ;
- des exemples de séquences de polyuréthanes selon l'invention, issus de composés polyols soufrés de formule (I) (Fig. 3) ;
- un schéma de synthèse possible d'un monomère polyol (Monomère M1 noté également ici A1) de formule (I) à partir de deux composés (Composé 1 et Composé 2) (Fig. 4.1), puis d'un polymère polyuréthane (Polymère P1) selon l'invention à partir du Monomère A1 et d'un monomère diisocyanate MDI (Monomère A2) (Fig. 4.2) ;
- un spectre RMN ¹H (500 MHz) respectivement du Monomère A1 et de son Composé 1 de départ, tous deux dissous dans CDCl₃ (Fig. 5.1 et Fig. 5.2) ;
- un autre schéma de synthèse possible, à partir du Monomère A1 et d'un autre monomère diisocyanate (Monomère A3, MDI bloqué benzophénone), du même Polymère P1 selon l'invention (Fig. 6) ;
- un schéma de synthèse possible d'un autre monomère polyol (Monomère M2 noté également ici A4) de formule (I) (Fig. 7.1), ainsi que d'un autre polyuréthane (Polymère P2) selon l'invention à partir de ce Monomère A4 et du Monomère A3 précédent (Fig. 7.2) ;
- un schéma de synthèse possible, à partir du Monomère A1 et de deux autres monomères (A5 et A3) d'un autre polyuréthane selon l'invention (Polymère P3) (Fig. 8).

### 4. DESCRIPTION DETAILLEE DE L'INVENTION

On rappellera tout d'abord ici qu'un polyuréthane est un polymère (par définition tout homopolymère ou copolymère, notamment copolymère à blocs) comportant une pluralité de liaisons uréthane (-O-CO-NH-) résultant de manière connue de la réaction d'addition d'un polyol ayant au moins deux fonctions alcool primaire, sur un polyisocyanate (composé porteur d'au moins deux fonctions isocyanate -NCO), notamment sur un diisocyanate dans le cas d'un polyuréthane du type linéaire.

Le polymère à unités uréthane conforme à l'invention est donc issu d'au moins un composé polyol (diol primaire) de formule (I) tel qu'énoncé en revendication 1.

Ce composé polyol soufré de formule (I) a donc notamment pour caractéristique essentielle de comporter un motif « M₁ » et le cas échéant (si « n » est égal à 1) un motif « M₂ » qui sont porteurs d'une part d'au moins une fonction alcool secondaire et d'autre part d'une fonction thioéther (-S-) en position α (alpha, c'est-à-dire pour rappel et par convention, portée par un carbone adjacent au carbone portant la fonction alcool secondaire) de cette fonction alcool.

Les formules génériques (II-1), (II-2) et (II-3) des motifs « M₁ » et « M₂ » sont représentées ci-dessous sous une forme développée :

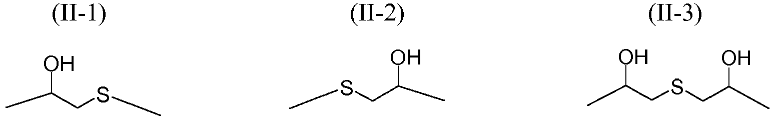

Dit autrement, le composé polyol dont est issu le polymère de l'invention a donc pour caractéristique de comporter au moins un (c'est-à-dire 1 ou plusieurs) motif α-hydroxy-thioéther. Dans le cas de la formule (II-3) où 2 fonctions alcool secondaire (et non une seule) sont situées de part et d'autre de la fonction thioéther, il s'agit d'un motif dénommé par convention α-α'-dihydroxy-thioéther.

Z₃ est soit (i) un groupement de liaison aliphatique ou cycloaliphatique, comportant une insaturation éthylénique, par exemple un groupe vinyle, soit (ii) un groupement aromatique comportant au moins un groupe phénylène, ce groupe phénylène pouvant être substitué ou non substitué.

Selon un mode de réalisation préférentiel, le groupement (cyclo) aliphatique comporte 3 à 30, plus préférentiellement 3 à 20 atomes de carbone.

Selon un autre mode de réalisation préférentiel, le groupement aromatique comporte 6 à 30, plus préférentiellement 6 à 20 atomes de carbone.

Selon un autre mode de réalisation préférentiel, Z₃ comporte au moins un (c'est-à-dire 1 ou plusieurs) hétéroatome choisi parmi O, S ou N.

Selon un autre mode de réalisation préférentiel, Z₃ comporte au moins une (c'est-à-dire 1 ou plusieurs) liaison éther (-O-) ou liaison thioéther (-S-), cette dernière pouvant être présente sur la chaîne carbonée (Z₃) elle-même ou sur un substituant d'un de ses atomes de carbone.

Sur les figures 1, 2.1 et 2.2 annexées ont été représentées, sous forme développée, les formules de trois exemples de composés polyols soufrés (aliphatiques ou aromatiques), tous de formule (I), notés Monomère M1 pour le polyol aliphatique et Monomères M2 et M3 pour les polyols aromatiques, ainsi qu'un schéma de synthèse utilisable pour l'obtention de chacun de ces composés.

Le composé polyol (Monomère M1, noté aussi par la suite Monomère A1) de la figure 1 est le 3-[3-(2-allyloxyméthyl-2-hydroxyméthyl-butoxy)-propylsulfanyl]-propane-1,2-diol. Il peut être obtenu par exemple par réaction de triméthylol-propane-diallyl éther et de thioglycérol, comme schématisé à la figure 1 ; cette synthèse a été décrite plus en détail dans les exemples de réalisation qui suivent (Essai 1 du paragraphe 5.1).

Le composé polyol (Monomère M2, noté aussi par la suite Monomère A4) de la figure 2.1 est le 3-{4-[4-(2,3-dihydroxy-propylsulfanyl)-phénylsulfanyl]-phénylsulfanyl}-propane-1,2-diol. Il peut être obtenu par exemple par réaction de 4,4'-thiobisbenzènethiol et de 3-chloro-1,2-propanediol liquide, comme schématisé à la figure 2.1. Sa synthèse est décrite plus en détail dans les exemples de réalisation qui suivent (Essai 5 du paragraphe 5.5).

Le composé polyol (Monomère M3) de la figure 2.2 est le 3-(4-{4-[4-(2,3-dihydroxy-propylsulfanyl)-phényl]-6-phényl-[1,3,5]triazin-2-yl}-phénylsulfanyl)-propane-1,2-diol.

Il peut être obtenu par exemple selon le procédé schématisé à la figure 2.2. Sous courant d'azote, on introduit dans un ballon 4 cols de 500 ml (muni d'un réfrigérant, d'un barreau magnétique et d'une entrée d'azote, préalablement séché sous vide à une température supérieure à 100°C), 5,70 g (soit 16,5 mmol) de 2,4-bis-(p-fluorophényl)-[1,3,5]-s-triazine, 200 ml de solvant DMSO anhydre, puis du carbonate de potassium sec (6,03 g soit 43,6 mmol) ; préalablement, le composé triazine et le carbonate de potassium ont été tous deux séchés sous vide à 150°C durant toute une nuit. Le composé triazine de départ a été synthétisé de manière connue, comme enseigné dans la demande WO 2012/016777 (Essai V-1-A, Composé 1, Fig. 18). Le système est ensuite purgé durant 30 min sous agitation et sous courant d'azote. On introduit alors 4,29 g (soit 39,6 mmol) de 1-thioglycérol puis la suspension est chauffée à 100°C durant 4,5 h sous courant d'azote et agitation. Le mélange réactionnel est ensuite refroidi à température ambiante (23°C) et le carbonate de potassium est éliminé par filtration sur papier filtre ; le sel est lavé avec 50 ml additionnels de DMSO. Le filtrat est ensuite versé dans 250 ml d'eau glacée et ainsi précipité. On ajoute alors 100 ml de solution de HCl (à 10%) et 1 litre d'eau déminéralisée ; le pH est ajusté à 1,0 à l'aide de la solution HCl (à 10%). Le précipité blanc (Monomère M3) ainsi obtenu (8,6 g, rendement d'environ 100%) est isolé par filtration sur papier filtre, lavé avec de l'eau déminéralisée et séché toute la nuit, à 90°C sous vide.

La caractéristique commune des trois composés polyols soufrés décrits précédemment aux figures 1 et 2, qu'ils soient aliphatiques ou aromatiques, est qu'ils répondent bien tous à la formule (I) :

HO-Z₁-M₁-Z₃-(M₂-Z₂)ₙ-OH

dans laquelle, pour ces exemples :
- Z₁ et le cas échéant Z₂ représentent tous un groupement méthylène, sauf pour le polyol de la Fig. 1.4 dans lequel Z₁ et Z₂ représentent l'éthylène ;
- Z₃ représente un groupement de liaison divalent répondant à la définition déjà donnée, selon les cas aliphatique (Fig. 1) ou aromatique (Fig. 2.1 et 2.2), mais comportant toujours au moins 3 atomes de carbone et pouvant comporter en outre une liaison éther (-O-) (Fig. 1) ou thioéther (-S-) (Fig. 2.1, ou un autre hétéroatome (N) (Fig. 2.2) ;
- « n » est un nombre entier égal à 0 (Fig. 1) ou à 1 (Fig. 2.1 et 2.2) ;
- les motifs « M₁ » et (le cas échéant) « M₂ » répondent à l'une des formules :

   (II-1) -CH(OH)-CH₂-S-

   (II-2) -S-CH₂-CH(OH)-

   (II-3) -CH(OH)-CH₂-S-CH₂-CH(OH)-.

Le composé polyol soufré de formule (I) précédemment décrit est préférentiellement utilisé pour la synthèse d'un polyuréthane conforme à l'invention du type linéaire, donc résultant essentiellement de l'addition de ce polyol, diol primaire, et d'un composé diisocyanate. Le diisocyanate utilisable peut être aromatique, aliphatique ou cycloaliphatique ; il peut s'agir d'un monomère, d'un prépolymère ou quasi-prépolymère, voir même d'un polymère.

Selon un mode de réalisation préférentiel, le diisocyanate dont est issu le polymère de l'invention est choisi dans le groupe constitué par les composés aromatiques suivants : diphénylméthane diisocyanate (en abrégé « MDI »), toluène diisocyanate (« TDI »), naphtalène diisocyanate (« NDI »), 3,3'-bitoluène diisocyanate (« TODI »), paraphénylène diisocyanate (« PPDI »), leurs différents isomères, et les mélanges de ces composés et/ou isomères.

Plus préférentiellement, on utilise un MDI ou un TDI, plus préférentiellement encore un MDI.

Tous les isomères du MDI (notamment 2,2'-MDI, 2,4'-MDI, 4,4'-MDI) et leurs mélanges sont utilisables, ainsi que des MDI dits polymériques (ou « PMDI ») comportant des oligomères de formule suivante (avec p égal ou supérieur à 1) :

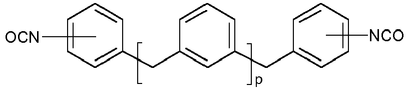

Sont également utilisables des composés diisocyanates du type aliphatiques, tels que par exemple 1,4-tétraméthylène diisocyanate, 1,6-hexane-diisocyanate (« HDI »), 1,4-bis(isocyanatométhyl)-cyclohexane, 1,3-bis(isocyanatométhyl)-cyclohexane, 1,3-bis(isocyanatométhyl)benzène, 1,4-bis(isocyanatométhyl)benzène, isophorone diisocyanate (« IPDI »), bis(4-iso-cyanatocyclohexyl)méthane diisocyanate (« H12MDI »), 4,4'-dicyclohexylméthane diisocyanate (« H13MDI »).

Selon un mode de réalisation particulièrement préférentiel, le diisocyanate utilisé est le 4,4'-MDI (4,4'-diphénylméthane diisocyanate) ayant pour formule : 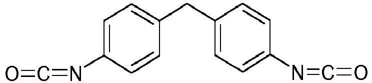 ou, si plusieurs diisocyanates sont utilisés, constitue le diisocyanate majoritaire en poids, représentant de préférence dans le dernier cas plus de 50% du poids total des composés diisocyanates.

On pourra aussi utiliser avantageusement un 4,4'-MDI bloqué caprolactame (par exemple le produit sous forme solide « Grilbond » IL-6 de la société EMS), de formule :

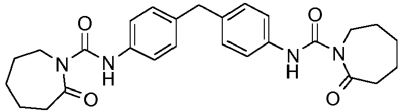

L'invention n'étant toutefois pas limitée à un polymère du type linéaire (pour rappel, issu d'un diisocyanate), on pourra également utiliser, notamment dans le but d'augmenter la Tg du polymère de l'invention par formation d'un réseau tridimensionnel, un composé triisocyanate tel que par exemple un trimère de MDI à noyau triazine de formule ci-dessous :

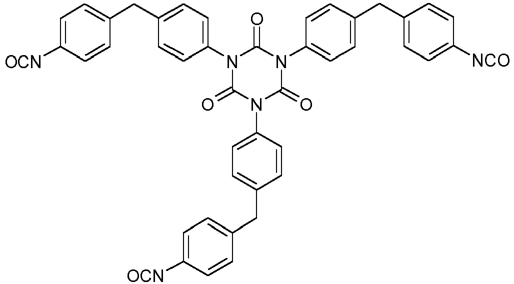

Le polyuréthane de l'invention a donc pour caractéristique de comporter, outre ses unités structurelles de base à unités uréthane (-O-CO-NH-) apportées de manière bien connue par le composé de départ polyisocyanate, des unités additionnelles spécifiques apportées par le monomère polyol de formule (I), ces unités additionnelles comportant au moins un motif (« M₁ ») ou deux motifs (« M₁ » et (« M₂ ») tels que décrits précédemment.

Les formules (III-1) à (III-3) ci-dessous reproduisent trois exemples de séquences (unités structurelles récurrentes) de polymères du type polyuréthane, ces séquences comportant d'une part des unités de base (uréthane) apportées par un diisocyanate (ici, de formule OCN-Z-NCO), et d'autre part des unités additionnelles apportées par un composé polyol de formule (I) (dans ce cas, « n » est égal à 0), ces unités additionnelles comportant au moins un motif « M₁ » de formules respectives II-1, II-2 et II-3 :

(III-1) -O-C(O)-NH-Z-NH-C(O)-O-Z₁-CH(OH)-CH₂-S-Z₃-

(III-2) -O-C(O)-NH-Z-NH-C(O)-O-Z₁-S-CH₂-CH(OH)-Z₃-

(III-3) -O-C(O)-NH-Z-NH-C(O)-O-Z₁-CH(OH)-CH₂-S-CH₂-CH(OH)-Z₃-

Dans les trois formules ci-dessus, Z représente un groupement de liaison au moins divalent, saturé ou insaturé, substitué ou non ; il s'agit de préférence d'un groupement aliphatique, cycloaliphatique ou aromatique, le groupement aliphatique comportant de préférence 1 à 30 (plus préférentiellement 1 à 20) atomes de carbone, le groupement cycloaliphatique de préférence de 3 à 30 (plus préférentiellement 3 à 20) atomes de carbone, le groupement aromatique de 6 à 30 (plus préférentiellement 6 à 20) atomes de carbone.

A titre d'exemples plus particuliers, la figure 3 annexée représente 3 exemples de formules développées de séquences de polyuréthanes selon l'invention répondant aux formules génériques (III-1) à (III-3) ci-dessus, dans lesquelles plus spécifiquement Z représente un résidu MDI et Z₁ représente un groupement méthylène.

Les figures 4 et 6 à 8 représentent en détail d'autres exemples préférentiels de polyuréthanes conformes à l'invention issus de polyols de formule (I), ainsi que divers schémas de synthèse possibles de ces polyuréthanes à partir de tels polyols.

Tout d'abord, les figures 4.1 et 4.2 illustrent respectivement des procédés de synthèse possibles d'un monomère polyol de formule (I) (noté Monomère A1), puis d'un polymère (noté Polymère P1) selon l'invention du type polyuréthane, à partir de ce Monomère A1 et d'un monomère diisocyanate MDI (Monomère A2), procédés qui seront décrits en détail ultérieurement.

Cet exemple de Polymère P1 comporte bien un motif récurrent de formule (III-1) :

-O-C(O)-NH-Z-NH-C(O)-O-Z₁-CH(OH)-CH₂-S-Z₃-

tel que défini précédemment, dans lequel Z correspond plus particulièrement au groupe divalent résidu de MDI, Z₁ est le méthylène, « M₁ » répond à la formule (II-1), et Z₃ représente un groupement aliphatique porteur d'une insaturation éthylénique (groupe vinyle).

On voit bien sur cette figure 4.2 que conformément à l'invention le Polymère P1 contient, en plus de ses unités de base uréthane, des unités additionnelles comportant une (seule) fonction α-hydroxy-thioéther.

La figure 6 illustre un autre procédé de synthèse possible de ce même Polymère P1 selon l'invention, cette fois à partir du Monomère A1 précédent et d'un autre monomère diisocyanate (Monomère A3, MDI bloqué caprolactame), procédé qui sera décrit en détail ultérieurement.

Les figures 7.1 et 7.2 illustrent des procédés de synthèse possible respectivement d'un autre monomère polyol de formule (I) (Monomère A4), puis d'un autre polymère (Polymère P2) conforme à l'invention du type polyuréthane, à partir du Monomère A3 et du Monomère A4, procédés qui seront décrits en détail ultérieurement.

Cet autre exemple de Polymère P2 préparé selon l'invention comporte bien un motif récurrent de formule :

-O-C(O)-NH-Z-NH-C(O)-O-Z₁-CH(OH)-CH₂-S-Z₃-S-CH₂-CH(OH)-Z₂-

dans lequel Z correspond plus particulièrement au groupe divalent résidu de MDI, Z₁ est le méthylène, « M₁ » répond à la formule (II-1) et « M₂ » répond à la formule (II-2), Z₃ représente un groupement aromatique porteur notamment d'une liaison thioéther (-S-) et Z₂ représente le méthylène.

On voit bien sur la figure 7.2 que conformément à l'invention le Polymère P2 comporte, en plus de ses unités de base uréthane, des unités additionnelles qui comportent cette fois, non pas une seule, mais deux fonctions α-hydroxy-thioéther.

La figure 8 illustre un autre procédé de synthèse d'un autre polymère (Polymère P3) conforme à l'invention, à partir des Monomère A1, Monomère A3 et d'un autre monomère polyol non conforme à la formule (I) (Monomère A5), procédé qui sera décrit en détail ultérieurement.

On voit bien sur la figure 8 que conformément à l'invention le Polymère P3 comporte, outre ses unités de base uréthane, des unités additionnelles qui comportent une fonction thioéther (-S-) en position α d'une fonction alcool secondaire (-CH(OH)-).

Le polyuréthane de l'invention synthétisable à partir d'un composé polyol soufré de formule (I) peut comporter de dix à plusieurs centaines, préférentiellement de 20 à 200 unités structurelles récurrentes telles décrites précédemment. Sa température de transition vitreuse Tg, mesurée par DSC (*Differential Scanning Calorimetry*), par exemple selon la norme ASTM D3418, est de préférence supérieure à 50°C, plus préférentiellement supérieure à 100°C, en particulier comprise entre 130°C et 250°C.

Ce polyuréthane présente une haute flexibilité, un allongement à la rupture important, il a révélé par ailleurs des propriétés hydrophobes et des propriétés anti-corrosion efficaces.

Il est avantageusement utilisable comme revêtement hydrophobe sur tout type de substrat, notamment en métal ou en verre, ou encore comme primaire d'adhésion sur tout type de renfort métallique, tel que par exemple un fil, un film, une plaque ou un câble en acier au carbone, laitonné ou non laitonné, destiné en particulier à renforcer une matrice de caoutchouc insaturé tel que du caoutchouc naturel.

### 5. EXEMPLES DE REALISATION DE L'INVENTION

Dans la présente demande, sauf indication expresse différente, tous les pourcentages (%) indiqués sont des pourcentages en masse.

### 5.1. Essai 1 - Synthèse du Monomère A1

Le monomère A1 (ou M1) est le 3-[3-(2-allyloxyméthyl-2-hydroxyméthyl-butoxy)-propylsulfanyl]-propane-1,2-diol, conforme à la formule (I). Ce monomère a été synthétisé selon le mode opératoire schématisé à la figure 4.1, comme détaillé ci-après : on place dans un ballon en verre de 50 ml, muni d'un barreau magnétique, 4,76 g de Composé 1 (triméthylol-propane-diallyl éther, pur à 90%, de Sigma Aldrich) puis 2,16 g de Composé 2 (thioglycérol), l'ensemble étant recouvert d'un bouchon en verre afin d'éviter des pertes par évaporation ; le mélange réactionnel est agité à température ambiante (20°C) durant 4 heures, puis toute la nuit suivante (environ 12 heures) à une température de 80°C.

On obtient ainsi un liquide visqueux transparent dont le spectre RMN (reproduit à la Fig. 5.1) comparé au spectre RMN du Composé 1 de départ (reproduit à la Fig. 5.2), en prenant comme référence la transition du groupe méthyle à 0,86 ppm (3 protons) et en suivant la diminution des intégrales correspondant aux protons du groupe vinyloxy, confirme bien à l'homme du métier qu'il s'agit du Monomère A1, de formule :

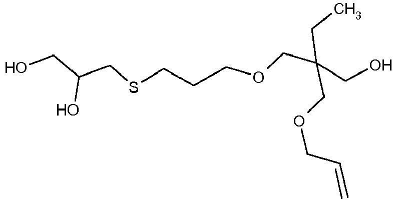

L'analyse RMN ¹H (500 MHz, CDCl₃) (Fig. 5.1) du produit a donné les résultats suivants : *0.85 (m, 3H), 1.39 (m, 2H), 11.86 (m, 1H), 2.65 (m, 3H),* 3.46 *m (8 H),* 3.73-3.75 (s, *2H),* 3.98 *(d, 2H), 5.16-5.19 (d, 1H), 5.28 (d, 1H), 5.88 (m, 1H).*

Enfin, la masse moléculaire du produit telle que mesurée par spectrométrie de masse "ESI" (*Electrospray Ionisation*), dans un mélange eau/acétonitrile 1/1 (avec traces de NaCl), a été évaluée en mode négatif (anion [M + Cl]⁻) à 357,3 (valeur théorique calculée égale à 357,5), et en mode positif (cation [M + Na]⁺) à 345,2 (valeur théorique calculée égale à 345,5).

### 5.2. Essai 2 - Synthèse du Polymère P1 par réaction des Monomères A1 et A2

Cet essai décrit de manière détaillée la synthèse du Polymère P1 selon l'invention, à partir des Monomères A1 et A2, selon le mode opératoire schématisé à la figure 4.2.

On place 3,40 g de Monomère A1 dans un ballon sec de 50 ml puis, à titre de catalyseur de polymérisation, 18,1 mg (soit 0,3% massique) de néodécanoate de bismuth et 100 ml de solvant γ-butyrolactone, le tout sous atmosphère inerte (courant d'azote). Puis, une solution (elle-même sous atmosphère inerte) de 2,64 g de Monomère A2 (MDI solide dissous dans 20 ml de γ-butyrolactone) est ajoutée à l'aide d'une ampoule à addition dans le ballon de 50 ml. Le mélange réactionnel transparent est agité et chauffé à 80°C durant 4 heures.

Puis on dépose 3 ml de la solution de Polymère P1 ainsi obtenu sur une plaque en verre (10x10 cm); la plaque de verre est entreposée sous vide et à 80°C durant 1 heure, jusqu'à ce que le solvant (γ-butyrolactone) soit évaporé. Le film transparent de Polymère P1 ainsi obtenu a été analysé par spectroscopie « ATR-FTIR » (*Attenuated Total Reflection InfraRed*) : la synthèse d'un polyuréthane est bien confirmée par l'apparition du pic visible à 1700 cm⁻¹, caractéristique de la liaison -OCONH-.

On a noté au passage que le Polymère P1 ainsi obtenu présentait une excellente adhésion au verre (impossibilité de séparer par traction le polymère du verre).

Puis, pour la mesure de sa masse moléculaire, le polymère dissous dans un mélange (1:20) de γ-butyrolactone et THF (tétrahydrofurane) a été soumis à une analyse GPC (*Gel Permeation Chromatography*) (colonne C18-phase inverse et THF comme phase mobile) : on a déterminé ainsi une masse moléculaire (Mw) d'environ 140 000 (témoins de polystyrène de masse moléculaire située entre 500 et 500 000). La même synthèse en l'absence du catalyseur de polymérisation a conduit à un profil très large d'élution avec une distribution majoritaire centrée à environ 17 000.

Enfin, la même synthèse conduite avec 1% en masse de catalyseur et dans le solvant DTP (1,3-diméthyl-3,4,5,6-tétrahydro-2(*1H*-pyrimidinone - CAS 7226-23-5) a conduit à une valeur de Tg égale à environ 93°C (DSC de -80°C à 200°C (10°C/min), 2^{ème} passage).

### 5.3. Essai 3 - Synthèse du Polymère P1 par réaction des Monomères A1 et A3

Le Polymère P1 conforme à l'invention a été également synthétisé à partir des monomères A1 et A3 (MDI bloqué caprolactame), selon le mode opératoire simple schématisé à la figure 6.

Dans un récipient en verre, on place 226,4 mg de Monomère A1 et 334,6 mg de Monomère A3 (« Grilbond » IL-6) ; puis 8 ml de solvant γ-butyrolactone sont ajoutés et le mélange est chauffé sous courant d'air chaud (120°C) jusqu'à l'obtention d'une solution claire.

3 ml de cette solution sont ensuite déposés de manière uniforme sur une plaque de verre (10x10 cm); le tout placée dans une étuve à 190°C pendant 15 min, sous vide afin d'éliminer les traces de solvant. Le film transparent ainsi obtenu de Polymère P1 a été caractérisé comme précédemment (ATR-FTIR) et a donné pratiquement le même spectre infrarouge. L'analyse DSC (second passage) de -80°C à 200°C (10°C/min) a révélé une Tg égale à environ 120°C.

### 5.4. Essai 4 - Test d'adhésion du Polymère 1 dans un composite métal / caoutchouc

Dans cet essai, un nouvel échantillon de Polymère P1 selon l'invention a été synthétisé comme indiqué dans l'essai précédent, en remplaçant simplement le solvant γ-butyrolactone par du DTP.

Un film fin du Polymère P1 ainsi obtenu a été déposé (à température ambiante) de manière uniforme à la surface d'une plaque de laiton. Puis on a recouvert le tout d'une couche de colle textile conventionnelle du type « RFL » (résorcinol-formaldéhyde-latex). Après un pré-séchage de 5 min à 100°C, l'ensemble a été ensuite traité à l'étuve à 190°C pendant 10 min.

La plaque en laiton ainsi revêtue du film de Polymère P1 et encollée RFL, a été ensuite disposée dans une matrice de composition (à l'état cru, non vulcanisé) de caoutchouc conventionnelle pour armature de ceinture de pneu tourisme, à base de caoutchouc naturel, de noir de carbone et silice à titre de charge, et d'un système de vulcanisation (soufre et accélérateur sulfénamide) ; cette composition étant dépourvue de sel de cobalt.

Puis l'éprouvette de composite métal / caoutchouc ainsi préparée a été placée sous presse et le tout cuit (vulcanisé) à 165°C pendant 30 min sous une pression de 20 bar. Après vulcanisation du caoutchouc, on a obtenu un excellent collage entre la matrice de caoutchouc et la plaque de métal malgré l'absence de sel de cobalt dans la matrice de caoutchouc : lors de tests de pelage conduits à la fois à température ambiante (23°C) et à haute température (100°C), on a constaté en effet que la rupture se produisait systématiquement dans la matrice de caoutchouc elle-même et non à l'interphase entre métal et caoutchouc.

### 5.5. Essai 5 - Synthèse du Monomère A4

Le monomère A4 (ou M2) est le 3-{4-[4-(2,3-dihydroxy-propylsulfanyl)-phénylsulfanyl]-phénylsulfanyl}-propane-1,2-diol, conforme à la formule (I). Ce monomère a été synthétisé selon le mode opératoire schématisé à la figure 7.1, comme détaillé ci-après.

Dans un ballon à 4 cols de 500 ml préalablement séché sous vide à plus de 100°C, équipé d'un condenseur, thermomètre et barreau magnétique, sont introduits sous atmosphère inerte (courant d'azote), 8,3 g de 4,4'-thiobisbenzènethiol solide (soit 33,03 mmol) puis 10,0 g de carbonate de potassium (soit 72,56 mmol, préalablement séché sous vide à 150°C pendant 12 h). On verse ensuite 200 ml de DMSO anhydre, la dispersion est purgée sous azote durant 30 min, puis on ajoute goutte à goutte 7,29 g de 3-chloro-1,2-propanediol liquide (soit 66,05 mmol). Le mélange réactionnel est chauffé immédiatement à 100°C durant 5 h, toujours sous courant azote. On laisse ensuite refroidir la solution obtenue à température ambiante, et le carbonate de potassium (K₂CO₃) solide est récupéré par filtrage sur un papier filtre. Le filtrat est ensuite lavé avec 50 ml de DMSO, puis le tout versé dans 250 ml d'eau déionisée à 0°C (glace) pour précipitation du produit visé.

Pour neutralisation du K₂CO₃ résiduel, on ajoute ensuite 10 ml de HCl (à 10%) et 1 litre d'eau déionisée. Après séchage sous vide à 60°C durant toute la nuit (12 h), le précipité obtenu (solide collant de couleur orange) est versé dans 500 ml d'acétate d'éthyle, le tout chauffé jusqu'à 40°C jusqu'à la dissolution complète. La solution obtenue est refroidie à température ambiante (20°C) puis transvasée dans une ampoule à séparation, 50 ml de NH4Cl saturé (aqueux) sont ensuite ajoutés. Après agitation, la phase organique est séparée puis lavée deux fois avec 25 ml d'eau déionisée, puis séchée avec du carbonate de sodium sec. Après filtration et distillation de l'acétate d'éthyle à l'évaporateur rotatif (« Rotavap »), le solide obtenu est séché à 80°C.

Les analyses RMN et ESI confirment bien la structure du Monomère A4, de formule :

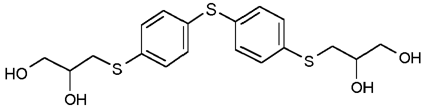

L'analyse RMN ¹H (500 MHz, DMSO-*d₆*) du produit a donné les résultats suivants :
*2.89 (m, 2H), 3.11 (m, 2H), 3.40 (m, 4H), 3.61 (m, 2H), 4.67 (d, 2H), 4.99 (d, 2H), 7.22-7.24 (d, 4H), 7.31-7.33 (d, 4H).*

Quant à la masse moléculaire mesurée par spectrométrie de masse ESI, dans un mélange acétate d'éthyle/méthanol (1/1) (avec traces de NaCl aqueux), elle a été évaluée en mode négatif (anion [M + Cl]⁻) à 433,3 (valeur théorique calculée égale à 434,0), et en mode positif (cation [M + Na]⁺) à 421,1 (valeur théorique calculée égale à 420,6).

### 5.6. Essai 6 - Synthèse du Polymère P2 par réaction des Monomères A3 et A4

Cet essai décrit de manière détaillée la synthèse du Polymère P2 conforme à l'invention, à partir des monomères A3 et A4, selon le mode opératoire schématisé à la figure 7.2.

On place dans un flacon en verre, 280,0 mg de Monomère A4, 334,6 mg de Monomère A3 et 8 ml de solvant NMP. La suspension est chauffée (sous courant d'air chaud) à 120°C, jusqu'à l'obtention d'une solution claire.

Puis on dépose 3 ml de la solution ainsi obtenue sur une plaque en laiton polie (10x10 cm) qui est ensuite passée à l'étuve à 190°C durant 15 min. On obtient ainsi un film transparent de Polymère P2 dont l'analyse DSC (second passage, de -80°C à 200°C ; 10°C/min) a révélé une Tg d'environ 115°C. On peut noter qu'une réaction de polymérisation similaire, mais conduite dans un mélange solvant 1-méthoxypropanol-2-acetate / sulfolane (3:1), a conduit à une Tg d'environ 130°C.

Un test d'adhésion du Polymère 2 dans un composite métal (plaque laiton) / caoutchouc, comme décrit à l'essai 4 qui précède, a lui aussi conduit à un excellent résultat, avec une rupture systématique dans la matrice de caoutchouc elle-même et non à l'interphase entre métal et caoutchouc.

### 5.7. Essai 7 - Synthèse du Polymère P3 par réaction des Monomères A1, A3 et A5

Cet essai décrit la synthèse du Polymère P3 conforme à l'invention, à partir des Monomères A1, A3 et A5, selon le mode opératoire schématisé à la figure 8.

Dans un flacon en verre, on verse successivement 1,34 g de monomère A1, 0,61 g de Monomère A5 (BHBA, acide 2,2-bis(hydroxyméthyl) propionique) puis 7,90 g de Monomère A3 (MDI bloqué caprolactame, « Grilbond » IL-6 50%-F). La suspension est agitée sous vibration mécanique (dispositif vortex) tout en augmentant légèrement la température jusqu'à environ 50°C (courant d'air chaud).

Puis 2,3 g de la suspension obtenue sont répartis de manière homogène sur une plaque en verre (10x10 cm) qui est ensuite traitée à l'étuve 10 min à 190°C, jusqu'à l'apparition d'un film clair de couleur jaune ; on traite 10 min supplémentaires sous vide afin d'évacuer les composants gazeux (soit un traitement total de 20 min à 190°C). On obtient ainsi un mince film jaune de Polymère P3 selon l'invention qui adhère très bien au verre (impossibilité de séparer par traction le polymère du verre).

On a également disposé 150 mg de la suspension ci-dessus sur une plaque de laiton (3x3 cm) ensuite traitée à l'étuve à 190°C durant 10 min, puis 10 min supplémentaires sous vide (soit un traitement total de 20 min à 190°C) ; on a pu confirmer une excellente adhésion du polymère selon l'invention également sur le métal, avec impossibilité de séparer par traction le polymère de la plaque de laiton.

En conclusion, les composés polyols soufrés de formule (I) permettent de synthétiser des polyuréthanes conformes à l'invention qui se caractérisent par une température de transition vitreuse élevée, une haute stabilité thermique et chimique, et une excellente adhésion au verre ou au métal.

Grâce à l'invention, ces polyuréthanes, utilisés comme primaire d'adhésion sur métal dans des composites métal / caoutchouc, permettent très avantageusement de coller ensuite le métal aux matrices de caoutchouc en utilisant de simples colles textiles telles que des colles « RFL » (résorcinol-formaldéhyde-latex) ou autres compositions adhésives équivalentes, ou encore directement (c'est-à-dire sans emploi de telles colles) à ces matrices de caoutchouc lorsque ces dernières contiennent par exemple des élastomères insaturés fonctionnalisés appropriés tels que des élastomères époxydés. Ainsi peuvent être notamment supprimés les sels de cobalt (ou autres sels métalliques) dans les compositions de caoutchouc destinées à être reliées à des renforts métalliques laitonnés.

## Revendications

1. Polymère à unités uréthane issu d'au moins un composé polyol soufré répondant à la formule :
(I) HO-Z₁-M₁-Z₃-(M₂-Z₂)ₙ-OH
dans laquelle:
- Z₁ et Z₂, identiques ou différents, représentent un groupement méthylène ou éthylène ;
- « n » est un nombre entier égal à 0 ou 1 ;
- les motifs « M₁ » et « M₂ », identiques ou différents, répondent à l'une des formules (II) ci-après :
(II-1) -CH(OH)-CH₂-S-
(II-2) -S-CH₂-CH(OH)-
(II-3) -CH(OH)-CH₂-S-CH₂-CH(OH)- ;
- Z₃, groupement de liaison au moins divalent comportant au moins 3 atomes de carbone, représente :
∘ soit un groupement aliphatique ou cycloaliphatique comportant une insaturation éthylénique ;
∘ soit un groupement aromatique comportant au moins un groupe phénylène.

2. Polymère selon la revendication 1, dans lequel Z₃ représente un groupement aliphatique ou cycloaliphatique comportant 3 à 30, de préférence 3 à 20 atomes de carbone.

3. Polymère selon la revendication 1, dans lequel Z₃ représente un groupement aromatique comportant 6 à 30, de préférence 6 à 20 atomes de carbone.

4. Polymère selon l'une quelconque des revendications 1 à 3, dans lequel Z₃ comporte un hétéroatome choisi parmi O, S ou N.

5. Polymère selon la revendication 4, dans lequel Z₃ comporte une liaison éther (-O-) ou thioéther (-S-).

6. Procédé de synthèse d'un polymère à unités uréthane, par polycondensation, avec un composé polyisocyanate, d'au moins un composé polyol soufré répondant à la formule :
(I) HO-Z₁-M₁-Z₃-(M₂-Z₂)ₙ-OH
dans laquelle:
- Z₁ et Z₂, identiques ou différents, représentent un groupement méthylène ou éthylène ;
- « n » est un nombre entier égal à 0 ou 1 ;
- les motifs « M₁ » et « M₂ », identiques ou différents, répondent à l'une des formules (II) ci-après :
(II-1) - CH(OH)-CH₂-S-
(II-2) - S-CH₂-CH(OH)-
(II-3) - CH(OH)-CH₂-S-CH₂-CH(OH)- ;
- Z₃, groupement de liaison au moins divalent comportant au moins 3 atomes de carbone, représente :
∘ soit un groupement aliphatique ou cycloaliphatique comportant une insaturation éthylénique ;
∘ soit un groupement aromatique comportant au moins un groupe phénylène.

7. Procédé selon la revendication 6, dans lequel Z₃ représente un groupement aliphatique ou cycloaliphatique comportant 3 à 30, de préférence 3 à 20 atomes de carbone.

8. Procédé selon la revendication 6, dans lequel Z₃ représente un groupement aromatique comportant 6 à 30, de préférence 6 à 20 atomes de carbone.

9. Procédé selon l'une quelconque des revendications 6 à 8, dans lequel Z₃ comporte un hétéroatome choisi parmi O, S ou N.

10. Procédé selon la revendication 9, dans lequel Z₃ comporte une liaison éther (-O-) ou thioéther (-S-).

11. Utilisation, pour la fabrication d'un polymère à unités uréthane, d'au moins un composé polyol soufré répondant à la formule :
(I) HO-Z₁-M₁-Z₃-(M₂-Z₂)ₙ-OH
dans laquelle:
- Z₁ et Z₂, identiques ou différents, représentent un groupement méthylène ou éthylène ;
- « n » est un nombre entier égal à 0 ou 1 ;
- les motifs « M₁ » et « M₂ », identiques ou différents, répondent à l'une des formules (II) ci-après :
(II-1) -CH(OH)-CH₂-S-
(II-2) -S-CH₂-CH(OH)-
(II-3) -CH(OH)-CH₂-S-CH₂-CH(OH)- ;
- Z₃, groupement de liaison au moins divalent comportant au moins 3 atomes de carbone, représente :
∘ soit un groupement aliphatique ou cycloaliphatique comportant une insaturation éthylénique ;
∘ soit un groupement aromatique comportant au moins un groupe phénylène.

12. Utilisation selon la revendication 11, dans lequel Z₃ représente un groupement aliphatique ou cycloaliphatique comportant 3 à 30, de préférence 3 à 20 atomes de carbone.

13. Utilisation selon la revendication 11, dans lequel Z₃ représente un groupement aromatique comportant 6 à 30, de préférence 6 à 20 atomes de carbone.

14. Utilisation selon l'une quelconque des revendications 11 à 13, dans lequel Z₃ comporte un hétéroatome choisi parmi O, S ou N.

15. Utilisation selon la revendication 14, dans lequel Z₃ comporte une liaison éther (-O-) ou thioéther (-S-).

## Patentansprüche

1. Polymer mit Urethaneinheiten, das sich aus mindestens einer schwefelhaltigen Polyolverbindung der folgenden Formel ergibt:
(I) HO-Z₁-M₁-Z₃-(M₂-Z₂)ₙ-OH
in der:
- Z₁ und Z₂ gleich oder verschieden sind und für eine Methylen- oder Ethylengruppe stehen;
- "n" eine ganze Zahl mit einem Wert von 0 oder 1 ist;
- die Einheiten "M₁" und "M₂" gleich oder verschieden sind und einer der nachstehenden Formeln (II) entsprechen:
(II-1) -CH(OH)-CH₂-S-
(II-2) -S-CH₂-CH(OH)-
(II-3) -CH(OH)-CH₂-S-CH₂-CH(OH)-;
- Z₃, eine mindestens zweiwertige Verbindungsgruppe mit mindestens 3 Kohlenstoffatomen, für:
∘ entweder eine aliphatische oder cycloaliphatische Gruppe mit einer ethylenischen Ungesättigtheit
∘ oder eine aromatische Gruppe mit mindestens einer Phenylengruppe
steht.

2. Polymer nach Anspruch 1, wobei Z₃ für eine aliphatische oder cycloaliphatische Gruppe mit 3 bis 30 und vorzugsweise 3 bis 20 Kohlenstoffatomen steht.

3. Polymer nach Anspruch 1, wobei Z₃ für eine aromatische Gruppe mit 6 bis 30 und vorzugsweise 6 bis 20 Kohlenstoffatomen steht.

4. Polymer nach einem der Ansprüche 1 bis 3, wobei Z₃ ein Heteroatom, das aus O, S oder N ausgewählt ist, enthält.

5. Polymer nach Anspruch 4, wobei Z₃ eine Etherbindung (-O-) oder Thioetherbindung (-S-) enthält.

6. Verfahren zur Synthese eines Polymers mit Urethaneinheiten durch Polykondensation mindestens einer schwefelhaltigen Polyolverbindung der Formel:
(I) HO-Z₁-M₁-Z₃-(M₂-Z₂)ₙ-OH
in der:
- Z₁ und Z₂ gleich oder verschieden sind und für eine Methylen- oder Ethylengruppe stehen;
- "n" eine ganze Zahl mit einem Wert von 0 oder 1 ist;
- die Einheiten "M₁" und "M₂" gleich oder verschieden sind und einer der nachstehenden Formeln (II) entsprechen:
(II-1) -CH(OH)-CH₂-S-
(II-2) -S-CH₂-CH(OH)-
(II-3) -CH(OH)-CH₂-S-CH₂-CH(OH)-;
- Z₃, eine mindestens zweiwertige Verbindungsgruppe mit mindestens 3 Kohlenstoffatomen, für:
∘ entweder eine aliphatische oder cycloaliphatische Gruppe mit einer ethylenischen Ungesättigtheit
∘ oder eine aromatische Gruppe mit mindestens einer Phenylengruppe
steht;
mit einer Polyisocyanatverbindung.

7. Verfahren nach Anspruch 6, wobei Z₃ für eine aliphatische oder cycloaliphatische Gruppe mit 3 bis 30 und vorzugsweise 3 bis 20 Kohlenstoffatomen steht.

8. Verfahren nach Anspruch 6, wobei Z₃ für eine aromatische Gruppe mit 6 bis 30 und vorzugsweise 6 bis 20 Kohlenstoffatomen steht.

9. Verfahren nach einem der Ansprüche 6 bis 8, wobei Z₃ ein Heteroatom, das aus O, S oder N ausgewählt ist, enthält.

10. Verfahren nach Anspruch 9, wobei Z₃ eine Etherbindung (-O-) oder Thioetherbindung (-S-) enthält.

11. Verwendung mindestens einer schwefelhaltigen Polyolverbindung der Formel:
(I) HO-Z₁-M₁-Z₃-(M₂-Z₂)ₙ-OH
in der:
- Z₁ und Z₂ gleich oder verschieden sind und für eine Methylen- oder Ethylengruppe stehen;
- "n" eine ganze Zahl mit einem Wert von 0 oder 1 ist;
- die Einheiten "M₁" und "M₂" gleich oder verschieden sind und einer der nachstehenden Formeln (II) entsprechen:
(II-1) -CH(OH)-CH₂-S-
(II-2) -S-CH₂-CH(OH)-
(II-3) -CH(OH)-CH₂-S-CH₂-CH(OH)-;
- Z₃, eine mindestens zweiwertige Verbindungsgruppe mit mindestens 3 Kohlenstoffatomen, für:
∘ entweder eine aliphatische oder cycloaliphatische Gruppe mit einer ethylenischen Ungesättigtheit
∘ oder eine aromatische Gruppe mit mindestens einer Phenylengruppe
steht;
zur Herstellung eines Polymers mit Urethaneinheiten.

12. Verwendung nach Anspruch 11, wobei Z₃ für eine aliphatische oder cycloaliphatische Gruppe mit 3 bis 30 und vorzugsweise 3 bis 20 Kohlenstoffatomen steht.

13. Verwendung nach Anspruch 11, wobei Z₃ für eine aromatische Gruppe mit 6 bis 30 und vorzugsweise 6 bis 20 Kohlenstoffatomen steht.

14. Verwendung nach einem der Ansprüche 11 bis 13, wobei Z₃ ein Heteroatom, das aus O, S oder N ausgewählt ist, enthält.

15. Verwendung nach Anspruch 14, wobei Z₃ eine Etherbindung (-O-) oder Thioetherbindung (-S-) enthält.

## Claims

1. Polymer having urethane units which results from at least one sulphur-comprising polyol compound corresponding to the formula :
(I) HO-Z₁-M₁-Z₃-(M₂-Z₂)ₙ-OH
in which:
- Z₁ and Z₂, which are identical or different, represent a methylene or ethylene group;
- "n" is an integer equal to 0 or 1;
- "M₁" and "M₂" units, which are identical or different, correspond to one of the formulae (II) below:
(II-1) -CH(OH)-CH₂-S-
(II-2) -S-CH₂-CH(OH)-
(II-3) -CH(OH)-CH₂-S-CH₂-CH(OH)-;
- Z₃, an at least divalent bonding group comprising at least 3 carbon atoms, represents:
∘ either an aliphatic or cycloaliphatic group comprising an ethylenic unsaturation;
∘ or an aromatic group comprising at least one phenylene group.

2. Polymer according to Claim 1, in which Z₃ represents an aliphatic or cycloaliphatic group comprising from 3 to 30, preferably from 3 to 20, carbon atoms.

3. Polymer according to Claim 1, in which Z₃ represents an aromatic group comprising from 6 to 30, preferably from 6 to 20, carbon atoms.

4. Polymer according to any one of Claims 1 to 3, in which Z₃ comprises a heteroatom chosen from O, S or N.

5. Polymer according to Claim 4, in which Z₃ comprises an ether (-O-) or thioether (-S-) bond.

6. Process for the synthesis of a polymer having urethane units, by polycondensation, with a polyisocyanate compound, of at least one sulphur-comprising polyol compound corresponding to the formula :
(I) HO-Z₁-M₁-Z₃-(M₂-Z₂)ₙ-OH
in which:
- Z₁ and Z₂, which are identical or different, represent a methylene or ethylene group;
- "n" is an integer equal to 0 or 1;
- "M₁" and "M₂" units, which are identical or different, correspond to one of the formulae (II) below:
(II-1) -CH(OH)-CH₂-S-
(II-2) -S-CH₂-CH(OH)-
(II-3) -CH(OH)-CH₂-S-CH₂-CH(OH)-;
- Z₃, an at least divalent bonding group comprising at least 3 carbon atoms, represents:
∘ either an aliphatic or cycloaliphatic group comprising an ethylenic unsaturation;
∘ or an aromatic group comprising at least one phenylene group.

7. Process according to Claim 6, in which Z₃ represents an aliphatic or cycloaliphatic group comprising from 3 to 30, preferably from 3 to 20, carbon atoms.

8. Process according to Claim 6, in which Z₃ represents an aromatic group comprising from 6 to 30, preferably from 6 to 20, carbon atoms.

9. Process according to any one of Claims 6 to 8, in which Z₃ comprises a heteroatom chosen from O, S or N.

10. Process according to Claim 9, in which Z₃ comprises an ether (-O-) or thioether (-S-) bond.

11. Use, for the manufacture of a polymer having urethane units, of at least one sulphur-comprising polyol compound corresponding to the formula :
(I) HO-Z₁-M₁-Z₃-(M₂-Z₂)ₙ-OH
in which:
- Z₁ and Z₂, which are identical or different, represent a methylene or ethylene group;
- "n" is an integer equal to 0 or 1;
- "M₁" and "M₂" units, which are identical or different, correspond to one of the formulae (II) below:
(II-1) -CH(OH)-CH₂-S-
(II-2) -S-CH₂-CH(OH)-
(II-3) -CH(OH)-CH₂-S-CH₂-CH(OH)-;
- Z₃, an at least divalent bonding group comprising at least 3 carbon atoms, represents:
∘ either an aliphatic or cycloaliphatic group comprising an ethylenic unsaturation;
∘ or an aromatic group comprising at least one phenylene group.

12. Use according to Claim 11, in which Z₃ represents an aliphatic or cycloaliphatic group comprising from 3 to 30, preferably from 3 to 20, carbon atoms.

13. Use according to Claim 11, in which Z₃ represents an aromatic group comprising from 6 to 30, preferably from 6 to 20, carbon atoms.

14. Use according to any one of Claims 11 to 13, in which Z₃ comprises a heteroatom chosen from O, S or N.

15. Use according to Claim 14, in which Z₃ comprises an ether (-O-) or thioether (-S-) bond.
